# FASCICULE DE BREVET EUROPEEN

(11) **EP 3 346 798 B1**
(45) Date de publication et mention de la délivrance du brevet: **17.06.2020**
(21) Numéro de dépôt: 18150002.6
(22) Date de dépôt: 01.01.2018
(51) Int. Cl.: H05B 1/02, A24F 47/00

(54) **UN ATOMISEUR DE CIGARETTE ÉLECTRONIQUE ÉQUIPÉ D'UNE PUCE D'ENREGISTREMENT ET UNE CIGARETTE ÉLECTRONIQUE ÉQUIPÉE DUDIT ATOMISEUR AINSI QUE SON PROCÉDÉ DE CONTRÔLE**
ZERSTÄUBER EINER ELEKTRONISCHEN ZIGARETTE, DER MIT EINEM SPEICHERCHIP AUSGESTATTET IST, UND EINE ELEKTRONISCHE ZIGARETTE, DIE MIT DIESEM ZERSTÄUBER AUSGESTATTET IST, SOWIE SEIN KONTROLLVERFAHREN
ELECTRONIC CIGARETTE ATOMISER PROVIDED WITH A RECORDING CHIP AND ELECTRONIC CIGARETTE PROVIDED WITH SAID ATOMISER AND THE METHOD FOR CONTROLLING SAME

(30) Priorité: 10.01.2017 CN 201710016022; 22.02.2017 FR 1751405
(43) Date de publication de la demande: 11.07.2018
(73) Titulaire: Shenzhen Innokin Technology Co., Ltd., Shenzhen Guangdong 518104 (CN)
(72) Inventeur: LI, Jian Wei, Shenzhen 518104 (CN)
(74) Mandataire: Hirsch & Partners

(56) Documents cités:
- EP-A1- 2 959 784
- WO-A2-2014/199233
- CN-U- 204 519 375
- CN-U- 205 567 823
- US-A1- 2013 284 192
- US-A1- 2016 174 076
- US-A1- 2017 000 192

## Description

### Domaine technique

La présente invention concerne le domaine technique de la fabrication des cigarettes électroniques, et en particulier un atomiseur de cigarette électronique équipé d'une puce d'enregistrement et une cigarette électronique équipée dudit atomiseur ainsi que son procédé de contrôle.

### Etat de la technique

La structure des cigarettes électronique comprend généralement un dispositif d'alimentation électrique de cigarette électronique, c'est-à-dire une source d'alimentation, un circuit de contrôle de cigarette électronique, c'est-à-dire un contrôleur de cigarette électronique, un dispositif d'alimentation en liquide aromatique pour la cigarette électronique, appelé cartouche en langage courant, un dispositif d'aspiration de la vapeur et un dispositif d'atomisation de liquide aromatique de cigarette électronique, c'est-à-dire un atomiseur de cigarette électronique. L'atomiseur de cigarette électronique est généralement constitué d'une chambre d'atomisation, la chambre d'atomisation est dotée d'un noyau de l'atomiseur destiné à atomiser le liquide aromatique de la cigarette électronique en le chauffant. Le noyau de l'atomiseur est doté de pôles positif et négatif connectés au circuit de contrôle de la cigarette électronique. Le noyau de l'atomiseur est généralement constitué d'une enveloppe externe et de fils chauffant internes, les fils chauffant internes sont en outre dotés d'un élément de transport et de stockage du liquide aromatique de la cigarette électronique par l'effet de perméation, tel que la bourre de coton, généralement conçue en forme de cordelette, reliée au réservoir de liquide aromatique de la cigarette électronique situé à l'extérieur via un orifice d'arrivée de liquide agencé à la paroi externe de l'enveloppe externe du noyau de l'atomiseur, afin de faciliter l'effet de perméation de la bourre de coton et d'amener le liquide aromatique aux fils chauffants du noyau de l'atomiseur et de l'atomiser en le chauffant. Les fils chauffants sont dotés de pôles positif et négatif respectivement connectés aux pôles positif et négatif de l'alimentation électrique via des fils conducteurs et colonnes conductrices.

Les bourres équipant les atomiseurs actuels et de ces dernières années sont majoritairement en coton ou en tissus non tissé, ils sont directement en contact avec les fils chauffants, la température de fonctionnement des fils chauffants est comprise entre 150 °C et 315 °C, la température peut être encore plus élevée s'ils sont secs. La résistance de la bourre de coton aux hautes températures est faible, ses températures de carbonisation et d'inflammation sont très faibles, en conditions normales d'alimentation en liquide aromatique, la durée de service de la bourre de coton est également limitée. Tout d'abord lorsqu'elle a été utilisée longuement, la bourre de coton perd son élasticité, ses fonctions de transport et de stockage de liquide aromatique chutent, ce qui engendre des fuites de l'atomiseur ainsi que des phénomènes de fuite de liquide aromatique dans le conduit de sortie, deuxièmement, la bourre de coton se carbonise lorsqu'elle a été longuement utilisée, du carbone s'accumule à la surface des fils chauffants, ce qui affecte le goût procuré à l'utilisateur et, plus grave, ceci est susceptible de produire des substances nocives ! Dans cet état, les noyaus d'atomiseur ne peuvent plus être utilisés, ils s'écartent totalement du concept de la cigarette électronique.

US2013284192 A1 concerne une cigarette électronique avec des améliorations de communication.

### Contenu de l'invention

Le problème technique à résoudre et l'effet technique à atteindre par la présente invention consistent à fournir un atomiseur de cigarette électronique équipé d'une puce d'enregistrement, permettant d'identifier de façon pratique des informations d'identification de l'atomiseur telles que les informations sur le liquide aromatique et le noyau de l'atomiseur, permettant d'enregistrer de façon continue des informations sur le fonctionnement de l'atomiseur telles que la durée de service et le nombre d'utilisations du noyau de l'atomiseur, afin de remplacer l'atomiseur, d'effectuer une recharge rationnelle en liquide aromatique, de maintenir l'atomiseur en état de fonctionnement normal, et d'éviter que la cigarette électronique fonctionne mal en raison d'un mauvais couplage ou d'une mauvaise utilisation.

Afin de résoudre les problèmes techniques et d'atteindre les effets techniques susmentionnés, un mode de réalisation de la présente invention fournit un atomiseur de cigarette électronique selon la revendication 1.

Préférablement, la puce d'enregistrement comprenant :
un premier enregistreur d'informations, destiné à enregistrer les informations d'identification de l'atomiseur ;
un deuxième enregistreur d'informations, destiné à enregistrer les informations de fonctionnement de l'atomiseur ;
un transmetteur d'informations, destiné à envoyer au contrôleur de cigarette électronique, les informations d'identification de l'atomiseur et les informations de fonctionnement de l'atomiseur obtenues au contrôleur de cigarette électronique enregistrées.

Préférablement, lorsque ledit atomiseur de cigarette électronique est doté d'une cartouche de liquide aromatique, ladite cartouche de liquide aromatique est dotée d'un premier enregistreur d'informations.

Préférablement, ledit premier enregistreur d'informations comprend un marqueur d'informations et un identificateur d'informations, ledit marqueur d'informations est connecté à ladite cartouche de liquide aromatique. Lorsqu'une cartouche de liquide aromatique est installée avec ledit atomiseur de cigarette électronique, ledit identificateur d'informations identifie ledit marqueur d'informations, acquiert les informations d'identification de la cartouche de liquide aromatique et, en fonction desdites informations d'identification de la cartouche de liquide aromatique , confirme et enregistre les informations du liquide aromatique et les informations de l'atomiseur.

Préférablement, ledit marqueur d'informations est un code-barres, un code QR ou une piste magnétique, ledit identificateur d'informations est un identificateur de code-barres, un identificateur de code QR ou un identificateur de piste magnétique, selon le cas.

Préférablement, ledit deuxième enregistreur d'informations est connecté audit noyau de l'atomiseur.

Le deuxième problème technique à résoudre et effet technique à atteindre par la présente invention consistent à fournir une cigarette électronique, permettant de coupler de façon adéquate et pratique liquide aromatique et atomiseur en fonction des informations d'identification de l'atomiseur enregistrées et d'ajuster les paramètres de fonctionnement du noyau de l'atomiseur, afin de coupler de façon rationnelle atomiseur et liquide aromatique, de maintenir l'atomiseur en état de fonctionnement normal, et d'éviter que la cigarette électronique fonctionne mal en raison d'un couplage ou d'une utilisation inadéquate de la cigarette électronique ; permettant de traiter les informations de fonctionnement de l'atomiseur telles que la durée de service et le nombre d'utilisations de l'atomiseur de cigarette électronique enregistrés, afin de contrôler à tout moment l'état de fonctionnement de la cigarette électronique, de stopper le fonctionnement de la cigarette électronique lorsque les informations de fonctionnement de l'atomiseur dépassent les valeurs seuil prédéfinies ou d'émettre un avertissement puis stopper le fonctionnement de la cigarette électronique lorsque les informations de fonctionnement de l'atomiseur approchent ou dépassent les valeurs seuil prédéfinies, afin d'éviter que l'atomiseur soit utilisé au-delà de sa durée de service, d'éviter que la bourre de coton se carbonise, et ainsi garantir l'homogénéité du produit et procurer une excellente expérience de vapotage à l'utilisateur.

Afin de résoudre les problèmes techniques et d'atteindre les effets techniques susmentionnés, un mode de réalisation de la présente invention fournit une cigarette électronique, comprenant l'atomiseur de cigarette électronique équipé de la puce d'enregistrement susmentionné et un contrôleur de cigarette électronique capable de recevoir et traiter les informations de l'atomiseur enregistrées, ledit contrôleur de cigarette électronique est conçu pour recevoir les informations d'identification de l'atomiseur enregistrées par ladite puce d'enregistrement, identifier et authentifier les informations d'identification de l'atomiseur reçues, contrôler le fonctionnement de l'atomiseur de cigarette électronique lorsque l'identification et l'authentification sont réussies, interrompre le fonctionnement de l'atomiseur de cigarette électronique lorsque l'identification et l'authentification échouent ; recevoir les informations de fonctionnement de l'atomiseur enregistrées par ladite puce d'enregistrement, déterminer si le noyau de l'atomiseur a dépassé sa durée de service, et stopper le fonctionnement de l'atomiseur de cigarette électronique lorsque le noyau de l'atomiseur a dépassé sa durée de service. Préférablement, ledit contrôleur de cigarette électronique comprend en outre :
un identificateur-authentificateur, destiné à recevoir les informations d'identification dudit atomiseur de cigarette électronique, à comparer et coupler les informations d'identification de l'atomiseur de cigarette électronique avec les informations d'identification de l'atomiseur de cigarette électronique prédéfinies, lorsque la comparaison et le couplage sont réussis, régler les paramètres de fonctionnement du noyau de l'atomiseur en fonction des informations d'identification de l'atomiseur de cigarette électronique reçues, lesdits paramètres de fonctionnement comprenant au moins la puissance de fonctionnement, le courant et la température du noyau de l'atomiseur, afin de contrôler le fonctionnement de l'atomiseur de cigarette électronique.

Préférablement, ledit contrôleur de cigarette électronique comprend en outre :
un contrôleur de dépassement de durée de service, destiné à acquérir les informations de fonctionnement de l'atomiseur, comparant les informations de fonctionnement de l'atomiseur avec les valeurs seuil de durées et/ou valeurs seuil de nombre d'utilisation prédéfinies, et interrompant le fonctionnement de l'atomiseur de cigarette électronique lorsque les informations de fonctionnement de l'atomiseur dépassent les valeurs seuil de durées et/ou valeurs seuil de nombre d'utilisations prédéfinies.

Préférablement, ladite cigarette électronique est doté d'un affichage, afin que lorsque les informations d'identification de l'atomiseur sont identifiées et authentifiées, les résultats de l'identification et de l'authentification soient affichés ; lorsque le noyau de l'atomiseur approche ou dépasse sa durée de service, il indique que le noyau de l'atomiseur approche ou a dépassé sa durée de service et qu'il faut le remplacer.

Un mode de réalisation de la présente invention fournit en outre un procédé de contrôle de l'atomiseur et de la cigarette électronique dotée dudit atomiseur susmentionné, ledit procédé comprend l'acquisition et l'enregistrement des informations d'identification de l'atomiseur, l'envoi des informations d'identification de l'atomiseur enregistrées au contrôleur de cigarette électronique ; la réception desdites informations d'identification de l'atomiseur, l'identification et l'authentification des informations d'identification de l'atomiseur reçues, le contrôle de fonctionnement de l'atomiseur de cigarette électronique lorsque l'identification et l'authentification sont réussies, l'arrêt du fonctionnement de l'atomiseur de cigarette électronique lorsque l'identification et l'authentification échouent ; l'acquisition et l'enregistrement des informations de fonctionnement de l'atomiseur enregistrées par la puce d'enregistrement, l'envoi desdites informations de fonctionnement de l'atomiseur au contrôleur de cigarette électronique ; la réception des informations de fonctionnement de l'atomiseur et la détermination du dépassement éventuel de la durée de service du noyau de l'atomiseur, et l'arrêt du fonctionnement de l'atomiseur de cigarette électronique lorsque le noyau de l'atomiseur a dépassé sa durée de service.

Les effets bénéfiques des solutions techniques susmentionnées de la présente invention sont les suivants :
Dans les modes de réalisation susmentionnés, grâce au paramétrage de la puce d'enregistrement, il est possible d'acquérir et d'envoyer les informations d'identification de l'atomiseur et les informations de fonctionnement de l'atomiseur de la cigarette électronique, telles que les informations sur le liquide aromatique, les informations sur le noyau de l'atomiseur, la durée de service et le nombre d'utilisation du noyau de l'atomiseur ; de recevoir et traiter, via le contrôleur de cigarette électronique, les informations d'identification de l'atomiseur de la cigarette électronique et les informations de fonctionnement de l'atomiseur enregistrées par la puce d'enregistrement, d'identifier et d'authentifier les informations du liquide aromatique et les informations du noyau de l'atomiseur, de contrôler le vieillissement du noyau de l'atomiseur en fonction des durées de fonctionnement et du nombre d'utilisations du noyau de l'atomiseur, d'éviter l'occurrence de fuites de l'atomiseur, de mauvais couplage avec le noyau de l'atomiseur et de problèmes de vieillissement du noyau de l'atomiseur, améliorant la sécurité de l'atomiseur de la cigarette électronique et l'expérience de l'utilisateur.

### Description des figures annexes

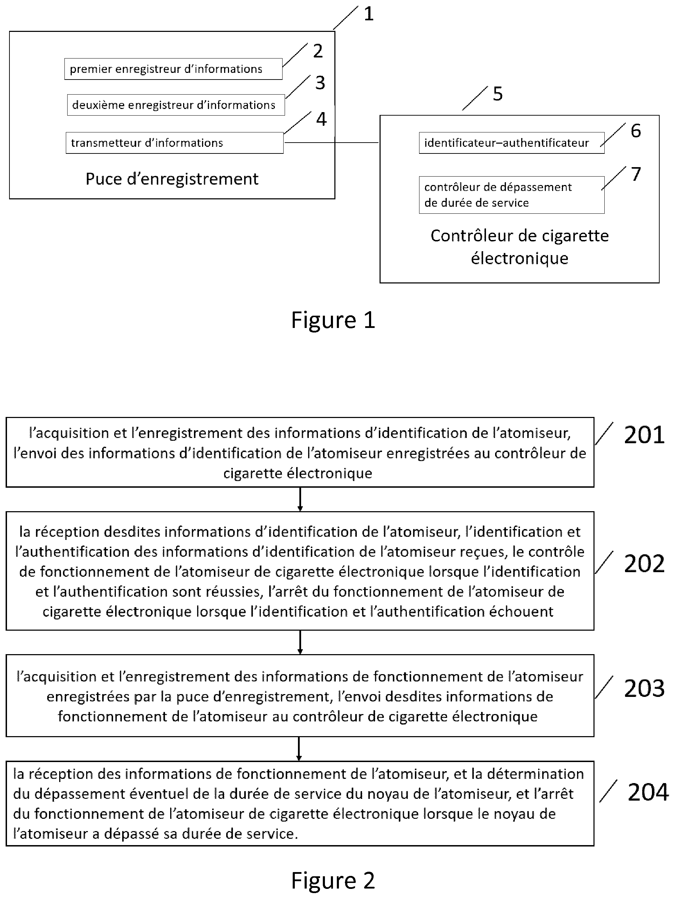

La Figure 1 représente le diagramme schématique de la puce d'enregistrement de l'atomiseur de la cigarette électronique de la présente invention réalisant l'enregistrement et l'envoi des informations de fonctionnement et d'identification de l'atomiseur de la cigarette électronique et le diagramme schématique du contrôleur de la cigarette électronique de la présente invention réalisant la réception et le traitement des informations de fonctionnement et d'identification de l'atomiseur de la cigarette électronique ;
la Figure 2 représente les étapes du procédé de contrôle de l'atomiseur de la cigarette électronique équipée de la puce d'enregistrement et du procédé de contrôle de la cigarette électronique comprenant ledit atomiseur.

### Mode de réalisation

Une description détaillée combinant figures annexes et mode de réalisation est donnée ci-dessous afin de clarifier le problème technique à résoudre, le plan technique et les avantages de la présente invention.

Le mode de réalisation de la présente invention, tel que représenté à la Figure 1, concerne un atomiseur de cigarette électronique équipé d'une puce d'enregistrement, , ladite puce d'enregistrement 1 est conçue pour acquérir et enregistrer les informations d'identification de l'atomiseur de cigarette électronique, lesdites informations d'identification comprenant au moins les informations sur le liquide aromatique et les informations sur le noyau de l'atomiseur de la cigarette électronique ; pour envoyer lesdites informations d'identification de l'atomiseur de cigarette électronique au contrôleur de cigarette électronique 5 ; pour enregistrer les informations de fonctionnement de l'atomiseur de cigarette électronique, lesdites informations de fonctionnement comprenant au moins la durée de service et le nombre d'utilisations du noyau de l'atomiseur ; et pour envoyer lesdites informations de fonctionnement de l'atomiseur au contrôleur de cigarette électronique 5.

Dans lequel, la puce d'enregistrement 1 comprend :
un premier enregistreur d'informations 2, destiné à acquérir et enregistrer les informations d'identification de l'atomiseur, lesdites informations comprenant au moins les informations sur le liquide aromatique et les informations sur le noyau de l'atomiseur ;
lesdites informations du liquide aromatique comprenant la composition du liquide aromatique et la quantité de liquide aromatique ;
lesdites informations du noyau de l'atomiseur comprenant le matériau des fils chauffants, les valeurs de résistances des fils chauffants et les informations concernant le liquide aromatique adapté au noyau de l'atomiseur, etc.

Un deuxième enregistreur d'informations 3, destiné à acquérir et enregistrer les informations de fonctionnement de l'atomiseur ; lesdites informations comprenant au moins la durée de service et le nombre d'utilisations du noyau de l'atomiseur ;
un transmetteur d'informations 4, destiné à envoyer au contrôleur de cigarette électronique les informations d'identification de l'atomiseur acquises et les informations de fonctionnement de l'atomiseur enregistrées.

Concrètement, la puce d'enregistrement 1 est agencée à l'intérieur de l'atomiseur de cigarette électronique (non présenté sur le dessin), elle peut être connectée au noyau de l'atomiseur, la puce d'enregistrement 1 peut être connectée aux deux pôles de l'atomiseur de cigarette électronique afin de l'alimenter, en outre elle est dotée d'un troisième pôle agencé sur l'atomiseur de cigarette électronique, et utilise la connexion du transmetteur d'informations avec le troisième pôle pour être connectée au contrôleur de cigarette électronique.

Le présent mode de réalisation fournit un premier enregistreur d'informations 2, un deuxième enregistreur d'informations 3 et un transmetteur d'informations 4, permettant d'acquérir rapidement et avec précision les informations du liquide aromatique, les informations du noyau de l'atomiseur, la durée de service et le nombre d'utilisations du noyau de l'atomiseur et de les envoyer au contrôleur de cigarette électronique 5, facilitant ainsi l'identification du liquide aromatique et du noyau de l'atomiseur par le contrôleur de cigarette électronique, le couplage rationnel du liquide aromatique et de l'atomiseur, et le contrôle de dépassement de la durée de service du noyau de l'atomiseur.

La présente invention fournit en outre une cigarette électronique comprenant l'atomiseur de cigarette électronique équipée de la puce d'enregistrement susmentionnée. L'intérieur de ladite cigarette électronique est doté d'un contrôleur de cigarette électronique 5, ledit contrôleur de cigarette électronique 5 est connecté au dispositif d'alimentation électrique de la cigarette électronique , c'est-à-dire une source d'alimentation comme la batterie, et contrôle les éléments de la cigarette électronique afin que celle-ci fonctionne normalement.

Dans le présent mode de réalisation, ledit contrôleur de cigarette électronique 5 sert également à recevoir les informations d'identification dudit atomiseur, c'est-à-dire au moins les informations du liquide aromatique et les informations du noyau de l'atomiseur, à identifier et authentifier les informations d'identification de l'atomiseur, à contrôler le fonctionnement de l'atomiseur de cigarette électronique lorsque l'identification et l'authentification sont réussies, à stopper le fonctionnement de l'atomiseur de cigarette électronique lorsque l'identification et l'authentification échouent ; à recevoir les informations de fonctionnement de l'atomiseur, c'est-à-dire au moins la durée de service et le nombre d'utilisations du noyau de l'atomiseur, à déterminer si le noyau de l'atomiseur a dépassé sa durée de service, et stopper le fonctionnement de l'atomiseur de cigarette électronique lorsque le noyau de l'atomiseur a dépassé sa durée de service.

Dans lequel, ledit contrôleur de cigarette électronique 5 comprend en outre :
un identificateur-authentificateur 6, destiné à recevoir les informations d'identification dudit atomiseur de cigarette électronique, à comparer et coupler les informations d'identification de l'atomiseur de cigarette électronique avec les informations d'identification de l'atomiseur de cigarette électronique prédéfinies, lorsque la comparaison et le couplage sont réussis, à régler les paramètres de fonctionnement du noyau de l'atomiseur en fonction des informations d'identification de l'atomiseur de cigarette électronique reçues, lesdits paramètres de fonctionnement comprenant au moins la puissance de fonctionnement, le courant et la température du noyau de l'atomiseur, afin de contrôler le fonctionnement de l'atomiseur de cigarette électronique.

Dans le présent mode de réalisation, le paramétrage de l'identificateur-authentificateur permet d'identifier rapidement et de façon pratique le liquide aromatique et le noyau de l'atomiseur, et ainsi d'évaluer le liquide aromatique et le noyau de l'atomiseur, en particulier de déterminer si le dispositif d'alimentation électrique du noyau de l'atomiseur est adapté, afin de fournir une alimentation électrique et un mode de fonctionnement adéquats.

Un contrôleur de dépassement de durée de service 7, destiné à acquérir les informations de fonctionnement de l'atomiseur, à comparer les informations de fonctionnement de l'atomiseur avec les valeurs seuil de durée de service et les valeurs seuil de nombre d'utilisations prédéfinies, et à stopper le fonctionnement de l'atomiseur de cigarette électronique lorsque les informations de fonctionnement de l'atomiseur dépassent les valeurs seuil de durée de service et/ou valeurs seuil de nombre d'utilisations prédéfinies, le contrôleur de dépassement de durée de service 7 peut également proposer le remplacement du noyau de l'atomiseur.

Dans le présent mode de réalisation, le contrôleur de dépassement de durée de service 7 est agencé pour contrôler efficacement la durée de service et le nombre d'utilisations du noyau de l'atomiseur afin d'éviter toute nuisance liée au vieillissement du noyau de l'atomiseur.

Préférablement, ledit agencement dispose d'un afficheur destiné à afficher les informations d'identification de l'atomiseur, c'est-à-dire que lorsque au moins les informations du liquide aromatique et les informations du noyau de l'atomiseur sont identifiées et authentifiées, les résultats de l'identification et de l'authentification sont affichés ; et à afficher les informations de fonctionnement de l'atomiseur, lorsque le noyau de l'atomiseur approche ou dépasse sa durée de service, il indique que le noyau de l'atomiseur approche ou a dépassé sa durée de service et qu'il faut le remplacer. Cette information est généralement envoyée avant que le fonctionnement de l'atomiseur de la cigarette électronique soit interrompu, il s'agit d'une information perceptive envoyée à l'utilisateur, par exemple incluant au moins une information visuelle, via un écran, un témoin lumineux ou une information sonore via un son d'avertissement, ou une information tactile via une vibration, etc.

Concrètement, lors de l'utilisation, l'atomiseur de la cigarette électronique est connecté au dispositif d'alimentation électrique de l'atomiseur, le contrôleur de cigarette électronique est connecté à la puce d'enregistrement, il enregistre et acquiert les informations d'identification de l'atomiseur, c'est-à-dire comprenant au moins les informations du liquide aromatique et les informations du noyau de l'atomiseur, lesdites informations du liquide aromatique comprennent la composition du liquide aromatique et la quantité de liquide aromatique ; lesdites informations du noyau de l'atomiseur comprennent le matériau des fils chauffants, les valeurs de résistances des fils chauffants et les informations concernant le liquide aromatique adapté au noyau de l'atomiseur, le contrôleur de cigarette électronique constitue le contrôleur central de la cigarette électronique pouvant contrôler l'affichage de ces informations sur l'afficheur (non représenté dans les figures) par le dispositif d'alimentation électrique de la cigarette électronique, lors du processus d'utilisation de l'atomiseur, la puce d'enregistrement accumule les informations de fonctionnement de l'atomiseur, et envoie les informations de nombre d'utilisation et de durée de service au contrôleur de cigarette électronique, lorsque le nombre d'utilisation et la durée de service dépassent les valeurs seuil prédéfinies, le contrôleur de la cigarette électronique stoppe l'alimentation électrique et propose le remplacement du noyau de l'atomiseur. Les valeurs seuil du nombre d'utilisation et de la durée de service peuvent être ajustés en fonction des besoins, ils peuvent être par exemple définis à 300 fois et 1000 heures. La proximité de la fin de la durée de service susmentionnée peut être établie lorsque les valeurs résiduelles de nombre d'utilisation et/ou de durée de service représentent 10 % ou 5 % des valeurs totales définies.

La puce d'enregistrement du présent mode de réalisation peut être conçue et choisie en fonction des besoins par l'homme du métier, l'homme du métier peut concevoir la forme concrète et l'emplacement de l'installation selon ses besoins, et la connecter avec le contrôleur de cigarette électronique en mode fil ou sans fil.

L'atomiseur de cigarette électronique équipé d'une puce d'enregistrement et la cigarette électronique équipée dudit atomiseur du présent mode de réalisation, peuvent, grâce à la configuration de la puce d'enregistrement permettant d'acquérir les informations d'identification de l'atomiseur et les informations de fonctionnement de l'atomiseur, c'est-à-dire au moins les informations du liquide aromatique, les informations du noyau de l'atomiseur, et les informations de durée de service et de nombre d'utilisations du noyau de l'atomiseur, identifier et authentifier de façon pratique les informations du liquide aromatique et les informations du noyau de l'atomiseur, et contrôler le vieillissement du noyau de l'atomiseur en fonction de la durée de service et du nombre d'utilisations du noyau de l'atomiseur, éviter l'occurrence de fuites de l'atomiseur, de mauvais couplage de noyau d'atomiseur et de problèmes de vieillissement du noyau de l'atomiseur, améliorant la sécurité de l'atomiseur de la cigarette électronique.

Les atomiseurs de cigarette électronique ordinaires comprennent généralement un couvercle supérieur d'atomiseur, une embase d'atomiseur, un cylindre externe d'atomiseur. Les atomiseurs de cigarette électronique jetables comprennent en outre une cartouche de liquide aromatique. Ledit couvercle supérieur d'atomiseur et ledit cylindre externe d'atomiseur sont connectés au sommet du cylindre externe d'atomiseur, ladite embase d'atomiseur et ledit cylindre externe d'atomiseur sont connectés à la partie inférieure du cylindre supérieur d'atomiseur. Les cartouches de liquide aromatique sont généralement agencées dans ledit cylindre externe d'atomiseur, une partie de ladite puce d'enregistrement peut être agencée sur la cartouche de liquide aromatique , la partie inférieure de ladite embase d'atomiseur est connectée au dispositif d'alimentation électrique de la cigarette électronique, ledit dispositif d'alimentation électrique de la cigarette électronique est doté d'un contrôleur de cigarette électronique, ladite puce d'enregistrement est connectée au contrôleur de cigarette électronique.

Dans le présent mode de réalisation, la facilitation de l'identification de la cartouche de liquide aromatique grâce à l'agencement d'une partie de la puce électronique sur la cartouche de liquide aromatique engendre la nécessité du couplage de la cartouche de liquide aromatique à l'atomiseur de cigarette électronique pour fonctionner, ce qui permet d'éviter l'endommagement de la cigarette électronique en raison d'une mauvaise utilisation de la cartouche de liquide aromatique .

La puce électronique comprend un premier enregistreur d'informations 2, destiné à acquérir et enregistrer les informations d'identification de l'atomiseur, un deuxième enregistreur d'informations 3, destiné à acquérir et enregistrer les informations de fonctionnement de l'atomiseur, et au moins la durée de service et le nombre d'utilisations du noyau de l'atomiseur ; un transmetteur d'informations 4, destiné à envoyer au contrôleur de cigarette électronique les informations d'identification de l'atomiseur de cigarette électronique acquises et les informations de fonctionnement de l'atomiseur enregistrées, c'est-à-dire au moins les informations du liquide aromatique, les informations du noyau de l'atomiseur, la durée de service et le nombre d'utilisations du noyau de l'atomiseur. Ledit premier enregistreur d'informations 2 comprend un marqueur d'informations et un identificateur d'informations, ledit marqueur d'informations est connecté à ladite cartouche de liquide aromatique , lorsqu'une cartouche de liquide aromatique est installée avec ledit atomiseur de cigarette électronique, ledit identificateur d'informations agencé sur l'atomiseur de cigarette électronique identifie ledit marqueur d'informations installé sur la cartouche de liquide aromatique, acquiert les informations d'identification de la cartouche de liquide aromatique et, en fonction desdites informations d'identification de la cartouche de liquide aromatique, confirme et enregistre les informations du liquide aromatique et les informations de l'atomiseur.

Dans le présent mode de réalisation, les dimensions de la puce d'enregistrement 1 sont relativement petites de sorte qu'elle peut être agencée sans problème dans l'atomiseur et connectée de façon pratique à la cartouche de liquide aromatique , la connexion à l'atomiseur est facilitée grâce à la connexion du contrôleur de cigarette électronique via le filetage de l'alimentation électrique à trois pôles.

Concrètement, la paroi externe ou la partie inférieure de la cartouche de liquide aromatique peuvent être dotées d'un code-barres, la paroi interne du cylindre externe de l'atomiseur ou la partie inférieure de l'atomiseur peuvent être dotées de l'identificateur de code-barres correspondant, lorsque la cartouche est installée dans l'atomiseur, l'identificateur de code-barres identifie le code-barres et acquiert les informations d'identification de l'atomiseur correspondant, un deuxième enregistreur d'informations lit et acquiert les informations de fonctionnement de l'atomiseur, le transmetteur d'informations envoie au contrôleur de cigarette électronique 5 les informations d'identification de l'atomiseur acquises et les informations de fonctionnement de l'atomiseur enregistrées.

Ou bien, la paroi externe ou la partie inférieure de la cartouche de liquide aromatique peuvent être dotées d'un code QR, la paroi interne du cylindre externe de l'atomiseur ou la partie inférieure de l'atomiseur peuvent être dotées de l'identificateur de code QR correspondant, lorsque la cartouche est installée dans l'atomiseur, l'identificateur de code QR identifie le code QR et acquiert les informations d'identification de l'atomiseur correspondant, un deuxième enregistreur d'informations lit et acquiert les informations de fonctionnement de l'atomiseur, le transmetteur d'informations envoie au contrôleur de cigarette électronique les informations d'identification de l'atomiseur acquises et les informations de fonctionnement de l'atomiseur enregistrées.

Dans le présent mode de réalisation, l'enregistrement des informations par code-barres et code QR permet de réduire l'espace occupé et de faciliter la lecture des informations.

Ou bien, la paroi externe ou la partie inférieure de la cartouche de liquide aromatique peuvent être dotées d'une piste magnétique, la paroi interne du cylindre externe de l'atomiseur ou la partie inférieure de l'atomiseur peuvent être dotées de l'identificateur de piste magnétique correspondant, lorsque la cartouche est installée dans l'atomiseur, l'identificateur de piste magnétique identifie la piste magnétique et acquiert les informations d'identification de l'atomiseur correspondant, un deuxième enregistreur d'informations lit et acquiert les informations de fonctionnement de l'atomiseur, les informations d'identification de l'atomiseur et les informations de fonctionnement de l'atomiseur sont envoyées au contrôleur de cigarette électronique.

Dans le présent mode de réalisation, l'utilisation du mode de piste magnétique pour l'enregistrement des informations permet d'accroître la sécurité et l'efficacité de la lecture des informations.

Préférablement ledit deuxième enregistreur d'informations 3 est connecté à ladite puce d'enregistrement 1.

En référence à la Figure 2, le procédé de contrôle de la cigarette électronique à atomiseur de cigarette électronique équipé de ladite puce d'enregistrement comprend :
une étape 201: l'acquisition et l'enregistrement des informations d'identification de l'atomiseur, et l'envoi desdites informations d'identification de l'atomiseur au contrôleur de cigarette électronique ;
une étape 202 : la réception des informations d'identification dudit atomiseur, l'identification et l'authentification des informations d'identification de l'atomiseur, ainsi qu'au moins les informations du liquide aromatique et les informations du noyau de l'atomiseur, le contrôle du fonctionnement de l'atomiseur de cigarette électronique lorsque l'identification et l'authentification sont réussies, et l'arrêt du fonctionnement de l'atomiseur de cigarette électronique lorsque l'identification et l'authentification échouent ;
une étape 203 : l'enregistrement des informations de fonctionnement de l'atomiseur, l'envoi lesdites informations de fonctionnement de l'atomiseur ainsi qu'au moins la durée de service et le nombre d'utilisations du noyau de l'atomiseur au contrôleur de cigarette électronique ;
une étape 204 : la réception des informations de fonctionnement de l'atomiseur, la détermination du dépassement éventuel de la durée de service par le noyau de l'atomiseur, et l'arrêt du fonctionnement de l'atomiseur de cigarette électronique lorsque le noyau de l'atomiseur a dépassé sa durée de service.

Préférablement, lors de la réception des informations d'identification dudit atomiseur de cigarette électronique, la comparaison et le couplage des informations d'identification de l'atomiseur, avec les informations d'identification de l'atomiseur de cigarette électronique prédéfinies, lorsque la comparaison et le couplage sont réussis, en fonction des informations d'identification de l'atomiseur de cigarette électronique reçues et envoyées par ladite puce d'enregistrement, le réglage de la puissance de fonctionnement, du courant et de la température du noyau de l'atomiseur, afin de contrôler le fonctionnement de l'atomiseur de cigarette électronique.

Préférablement, après réception des informations de fonctionnement de l'atomiseur de cigarette électronique reçues et envoyées par ladite puce d'enregistrement, la comparaison avec les valeurs seuil de durées et valeurs seuil de nombre d'utilisation prédéfinies correspondantes, lorsque les informations de fonctionnement de l'atomiseur approchent ou dépassent les valeurs seuil de durées et/ou valeurs seuil de nombre d'utilisations prédéfinies, l'arrêt du fonctionnement de l'atomiseur de cigarette électronique, le remplacement du noyau de l'atomiseur peut être affiché et/ou indiqué avant l'arrêt.

Le procédé de contrôle de l'atomiseur de cigarette électronique équipé d'une puce d'enregistrement du mode de réalisation de la présente invention est similaire aux spécificités du procédé de contrôle de la cigarette électronique à atomiseur de cigarette électronique équipé de la puce d'enregistrement susmentionnée, nous n'en donnerons pas de description supplémentaire.

Ce qui précède constitue un mode de réalisation préféré de la présente invention, il faut préciser que l'homme du métier peut, à la condition de ne pas s'écarter desdits principes de la présente invention, effectuer quelques améliorations et retouches, ces améliorations et retouches sont considérées comme couvertes par l'étendue de la protection de la présente invention.

## Revendications

1. Un atomiseur de cigarette électronique, ledit atomiseur de cigarette électronique comprend un noyau de l'atomiseur destiné à atomiser un liquide aromatique de la cigarette électronique,
ledit atomiseur de cigarette électronique est équipé d'une puce d'enregistrement (1), ladite puce d'enregistrement (1) est conçue pour :
- enregistrer les informations d'identification de l'atomiseur de cigarette électronique, lesdites informations d'identification comprenant au moins les informations sur le liquide aromatique et les informations sur le noyau de l'atomiseur, pour envoyer lesdites informations d'identification de l'atomiseur à un contrôleur (5) de cigarette électronique ; et
- enregistrer les informations de fonctionnement de l'atomiseur de cigarette électronique, lesdites informations de fonctionnement comprenant au moins la durée de service et le nombre d'utilisations du noyau de l'atomiseur, et pour envoyer lesdites informations de fonctionnement de l'atomiseur au contrôleur de cigarette électronique,
**caractérisé en ce que** lesdites informations du liquide aromatique comprennent la composition du liquide aromatique et la quantité de liquide aromatique , et **en ce que** lesdites informations du noyau de l'atomiseur comprennent le matériau des fils chauffants, les valeurs de résistance des fils chauffants et les informations concernant le liquide aromatique adapté au noyau de l'atomiseur.

2. L'atomiseur de cigarette électronique selon la revendication 1, **caractérisé en ce que** ladite puce d'enregistrement (1) comprenant :
- un premier enregistreur d'informations (2), destiné à enregistrer les informations d'identification de l'atomiseur ;
- un deuxième enregistreur d'informations (3), destiné à enregistrer les informations de fonctionnement de l'atomiseur ; et
- un transmetteur d'informations (4), destiné à envoyer au contrôleur (5) de cigarette électronique, les informations d'identification de l'atomiseur et les informations de fonctionnement obtenues de l'atomiseur.

3. L'atomiseur de cigarette électronique selon la revendication 2, **caractérisé en ce que** lorsque ledit atomiseur de cigarette électronique est doté d'une cartouche de liquide aromatique, ladite cartouche de liquide aromatique est dotée d'un premier enregistreur d'informations (2).

4. L'atomiseur de cigarette électronique selon la revendication 3, **caractérisé en ce que** ledit premier enregistreur d'informations (2) comprend un marqueur d'informations et un identificateur d'informations, ledit marqueur d'informations est conçu pour connecter à ladite cartouche de liquide aromatique, dans lequel, lorsqu'une cartouche de liquide aromatique est installée avec ledit atomiseur de cigarette électronique, ledit identificateur d'informations identifie ledit marqueur d'informations, acquiert les informations d'identification de la cartouche de liquide aromatique et, en fonction desdites informations d'identification de la cartouche de liquide aromatique, confirme et enregistre les informations du liquide aromatique et les informations de l'atomiseur.

5. L'atomiseur de cigarette électronique selon la revendication 4, **caractérisé en ce que** ledit marqueur d'informations est un code-barres, un code QR ou une piste magnétique, ledit identificateur d'informations est un identificateur correspondant.

6. L'atomiseur de cigarette électronique selon l'une quelconque des revendications 2 à 5, **caractérisé en ce que** ledit deuxième enregistreur d'informations (3) est connecté audit noyau de l'atomiseur.

7. Une cigarette électronique, **caractérisé en ce que** ladite cigarette électronique comprenant un atomiseur de cigarette électronique équipé de la puce d'enregistrement (1) selon l'une quelconque des revendications 1 à 6, et un contrôleur (5) de cigarette électronique conçu pour :
- recevoir les informations d'identification de l'atomiseur enregistrées par ladite puce d'enregistrement (1), identifier et authentifier les informations d'identification de l'atomiseur reçues, contrôler le fonctionnement de l'atomiseur de cigarette électronique lorsque l'identification et l'authentification sont réussies, interrompre le fonctionnement de l'atomiseur de cigarette électronique lorsque l'identification et l'authentification échouent ; et
- recevoir les informations de fonctionnement de l'atomiseur enregistrées par ladite puce d'enregistrement (1), déterminer si le noyau de l'atomiseur a dépassé sa durée de service, et stopper le fonctionnement de l'atomiseur de cigarette électronique lorsque le noyau de l'atomiseur a dépassé sa durée de service.

8. La cigarette électronique selon la revendication 7, **caractérisé en ce que** ledit contrôleur de cigarette électronique comprend en outre :
- un identificateur-authentificateur (6), destiné à recevoir les informations d'identification dudit atomiseur de cigarette électronique, à comparer et coupler les informations d'identification de l'atomiseur de cigarette électronique avec les informations d'identification de l'atomiseur de cigarette électronique prédéfinies, lorsque la comparaison et le couplage sont réussis, régler les paramètres de fonctionnement du noyau de l'atomiseur en fonction des informations d'identification de l'atomiseur de cigarette électronique reçues, lesdits paramètres de fonctionnement comprenant au moins la puissance de fonctionnement, le courant et la température du noyau de l'atomiseur, afin de contrôler le fonctionnement de l'atomiseur de cigarette électronique.

9. La cigarette électronique selon la revendication 7 ou 8, **caractérisé en ce que** ledit contrôleur de cigarette électronique comprend en outre :
- un contrôleur de dépassement de durée de service (7), destiné à acquérir les informations de fonctionnement de l'atomiseur, comparant les informations de fonctionnement de l'atomiseur avec les valeurs seuil de durées et/ou valeurs seuil de nombre d'utilisation prédéfinies, et interrompant le fonctionnement de l'atomiseur de cigarette électronique lorsque les informations de fonctionnement de l'atomiseur dépassent les valeurs seuil de durées de service et/ou valeurs seuil de nombre d'utilisations prédéfinies.

10. La cigarette électronique selon la revendication 9, **caractérisé en ce que** ladite cigarette électronique est doté d'un afficheur, destiné à afficher les informations d'identification de l'atomiseur, lorsque les informations d'identification de l'atomiseur sont identifiées et authentifiées ; et à afficher les informations de fonctionnement de l'atomiseur, en indiquant le remplacement du noyau de l'atomiseur, lorsque le noyau de l'atomiseur approche ou dépasse sa durée de service.

11. La cigarette électronique selon la revendication 10, **caractérisé en ce que** l'information de fonctionnement de l'atomiseur est envoyée avant que le fonctionnement de l'atomiseur de la cigarette électronique soit interrompu ; et que l'information de fonctionnement de l'atomiseur est une information perceptive envoyée à l'utilisateur incluant au moin : une information visuelle, via un écran, un témoin lumineux, une information sonore via un son d'avertissement, et une information tactile via une vibration.

12. Un procédé de contrôle de la cigarette électronique selon l'une quelconque des revendications 7 à 11, **caractérisé en ce que** ledit procédé comprend les étapes de :
- l'acquisition et l'enregistrement des informations d'identification de l'atomiseur, l'envoi des informations d'identification de l'atomiseur enregistrées au contrôleur de cigarette électronique (201);
- la réception desdites informations d'identification de l'atomiseur, l'identification et l'authentification des informations d'identification de l'atomiseur reçues, le contrôle de fonctionnement de l'atomiseur de cigarette électronique lorsque l'identification et l'authentification sont réussies, l'arrêt du fonctionnement de l'atomiseur de cigarette électronique lorsque l'identification et l'authentification échouent (202);
- l'acquisition et l'enregistrement des informations de fonctionnement de l'atomiseur enregistrées par la puce d'enregistrement, l'envoi desdites informations de fonctionnement de l'atomiseur au contrôleur de cigarette électronique (203);
- la réception des informations de fonctionnement de l'atomiseur, et la détermination du dépassement éventuel de la durée de service du noyau de l'atomiseur, et l'arrêt du fonctionnement de l'atomiseur de cigarette électronique lorsque le noyau de l'atomiseur a dépassé sa durée de service (204).

13. Le procédé de contrôle de la cigarette électronique selon la revendication 12, **caractérisé en ce que** ledit procédé comprend en outre, lors de la réception des informations d'identification dudit atomiseur de cigarette électronique, la comparaison et le couplage des informations d'identification de l'atomiseur avec les informations d'identification de l'atomiseur de cigarette électronique prédéfinies, lorsque la comparaison et le couplage sont réussis, en fonction des informations d'identification de l'atomiseur de cigarette électronique reçues et envoyées par ladite puce d'enregistrement, le réglage de la puissance de fonctionnement, du courant et de la température du noyau de l'atomiseur, afin de contrôler le fonctionnement de l'atomiseur de cigarette électronique.

14. Le procédé de contrôle de la cigarette électronique selon la revendication 12 ou 13, **caractérisé en ce que** ledit procédé comprend en outre, après réception des informations de fonctionnement de l'atomiseur de cigarette électronique reçues et envoyées par ladite puce d'enregistrement, la comparaison avec les valeurs seuil de durées et valeurs seuil de nombre d'utilisation prédéfinies correspondantes, lorsque les informations de fonctionnement de l'atomiseur approchent ou dépassent les valeurs seuil de durées et/ou valeurs seuil de nombre d'utilisations prédéfinies, l'arrêt du fonctionnement de l'atomiseur de cigarette électronique, le remplacement du noyau de l'atomiseur est indiqué avant l'arrêt.

## Patentansprüche

1. Ein Verdampfer für E-Zigaretten, wobei der Verdampfer für E-Zigaretten einen Verdampferkern aufweist, der dazu bestimmt ist, eine aromatische Flüssigkeit der E-Zigarette zu verdampfen, wobei der Verdampfer für E-Zigaretten mit einem Aufzeichnungschip (1) ausgestattet ist, wobei der Aufzeichnungschip (1) ausgelegt ist, um:
- die Identifikationsinformationen des Verdampfers für E-Zigaretten aufzuzeichnen, wobei die Identifikationsinformationen mindestens die Information über die aromatische Flüssigkeit und die Informationen über den Verdampferkern aufweisen, um die Identifikationsinformationen des Verdampfers an eine Steuerung (5) der E-Zigarette zu senden, und
- die Betriebsinformationen des Verdampfers für E-Zigaretten aufzuzeichnen, wobei die Betriebsinformationen mindestens die Betriebsdauer und die Anzahl der Verwendungen des Verdampferkerns aufweisen, und um die Betriebsinformation des Verdampfers an die Steuerung der E-Zigarette zu senden,
**dadurch gekennzeichnet, dass** die Informationen der aromatischen Flüssigkeit die Zusammensetzung der aromatischen Flüssigkeit und die Menge der aromatischen Flüssigkeit aufweisen und dadurch, dass die Informationen des Verdampferkerns das Material der Heizdrähte, die Widerstandswerte der Heizdrähte und die Informationen in Bezug auf die aromatische Flüssigkeit aufweisen, die für den Kern des Verdampfers geeignet ist.

2. Der Verdampfer für E-Zigaretten nach Anspruch 1, **dadurch gekennzeichnet, dass** der Aufzeichnungschip (1) aufweist:
- einen ersten Informationsaufzeichner (2), der dazu bestimmt ist, die Identifikationsinformation des Verdampfers aufzuzeichnen,
- einen zweiten Informationsaufzeichner (3), der dazu bestimmt ist, die Betriebsinformation des Verdampfers aufzuzeichnen, und
- einen Informationssender (4), der dazu bestimmt ist, an die Steuerung (5) der E-Zigarette die Identifikationsinformationen des Verdampfers und die Betriebsinformationen, die vom Verdampfer erhalten werden,.

3. Der Verdampfer für E-Zigaretten nach Anspruch 2, **dadurch gekennzeichnet, dass**, wenn der Verdampfer für E-Zigaretten mit einer Patrone für die aromatische Flüssigkeit ausgestattet ist, die Patrone für die aromatische Flüssigkeit mit einem ersten Informationsaufzeichner (2) ausgestattet ist.

4. Der Verdampfer für E-Zigaretten nach Anspruch 3, **dadurch gekennzeichnet, dass** der erste Informationsaufzeichner (2) einen Informationsmarker und einen Informationsidentifikator aufweist, wobei der Informationsmarker zum Anschließen an die Patrone für die aromatische Flüssigkeit ausgebildet ist, wobei, wenn die Patrone für die aromatische Flüssigkeit mit dem Verdampfer für E-Zigaretten installiert ist, der Informationsidentifikator den Informationsmarker identifiziert, die Identifikationsinformationen der Patrone für die aromatische Flüssigkeit erwirbt und in Abhängigkeit von den Identifikationsinformationen der Patrone für die aromatische Flüssigkeit die Informationen der aromatischen Flüssigkeit und die Informationen des Verdampfers bestätigt und aufzeichnet.

5. Der Verdampfer für E-Zigaretten nach Anspruch 4, **dadurch gekennzeichnet, dass** der Informationsmarker ein Strichcode, ein QR-Code oder ein Magnetstreifen ist, wobei der Informationsidentifikator ein entsprechender Identifikator ist.

6. Der Verdampfer für E-Zigaretten nach einem der Ansprüche 2 bis 5, **dadurch gekennzeichnet, dass** der zweite Informationsaufzeichner (3) an den Verdampferkern angeschlossen ist.

7. Eine E-Zigarette, **dadurch gekennzeichnet, dass** die E-Zigarette einen Verdampfer für E-Zigaretten, der mit dem Aufzeichnungschip (1) nach einem der Ansprüche 1 bis 6 ausgestattet ist, und eine Steuerung (5) der E-Zigarette aufweist, die ausgebildet ist, um:
- die Identifikationsinformationen des Verdampfers zu empfangen, die von dem Aufzeichnungschip (1) aufgezeichnet werden, die empfangenen Identifikationsinformationen des Verdampfers zu identifizieren und zu authentifizieren, den Betrieb des Verdampfers für E-Zigaretten zu steuern, wenn das Identifizieren und das Authentifizieren gelungen sind, den Betrieb des Verdampfers für E-Zigaretten zu unterbrechen, wenn das Identifizieren und das Authentifizieren scheitern und
- die Betriebsinformationen des Verdampfers zu empfangen, die von dem Aufzeichnungschip (1) aufgezeichnet werden, zu bestimmen, ob der Verdampferkern seine Betriebsdauer überschritten hat und den Betrieb des Verdampfers für E-Zigaretten zu stoppen, wenn der Verdampferkern seine Betriebsdauer überschritten hat.

8. Die E-Zigarette nach Anspruch 7, **dadurch gekennzeichnet, dass** die Steuerung der E-Zigarette ferner aufweist:
- einen Identifikator-Authentifikator (6), der dazu bestimmt ist, die Identifikationsinformation des Verdampfers für E-Zigaretten zu empfangen, die Identifikationsinformationen des Verdampfers für E-Zigaretten mit den vordefinierten Identifikationsinformationen des Verdampfers für E-Zigaretten zu vergleichen und zu verknüpfen, wenn das Vergleichen und das Verknüpfen gelungen sind, die Betriebsparameter des Verdampferkerns in Abhängigkeit von den erhaltenen Identifikationsinformationen des Verdampfers für E-Zigaretten einzustellen, wobei die Betriebsparameter mindestens die Betriebsleistung, den Strom und die Temperatur des Verdampferkerns aufweisen, um den Betrieb des Verdampfers für E-Zigaretten zu steuern.

9. Die E-Zigarette nach Anspruch 7 oder 8, **dadurch gekennzeichnet, dass** die Steuerung der E-Zigarette ferner aufweist:
- eine Steuerung der Überschreitung der Betriebsdauer (7), die dazu bestimmt ist, die Betriebsinformationen des Verdampfers zu erfassen, die die Betriebsinformationen des Verdampfers mit den vordefinierten Schwellenwerten der Dauern und/oder Schwellenwerten der Anzahl von Verwendungen vergleicht und den Betrieb des Verdampfers für E-Zigaretten unterbricht, wenn die Betriebsinformationen des Verdampfers die vordefinierten Schwellenwerte der Betriebsdauern und/oder Schwellenwerte der Anzahl der Verwendungen überschreiten.

10. Die E-Zigarette nach Anspruch 9, **dadurch gekennzeichnet, dass** die E-Zigarette mit einer Anzeige versehen ist, die dazu bestimmt ist, die Identifikationsinformationen des Verdampfers anzuzeigen, wenn die Identifikationsinformationen des Verdampfers identifiziert und authentifiziert sind, und die Betriebsinformationen des Verdampfers anzuzeigen, indem das Austauschen des Verdampferkerns angezeigt wird, wenn sich der Verdampferkern seiner Betriebsdauer nähert oder diese überschreitet.

11. Die E-Zigarette nach Anspruch 10, **dadurch gekennzeichnet, dass** die Betriebsinformation des Verdampfers gesendet wird, bevor der Betrieb des Verdampfers für E-Zigaretten unterbrochen wird, und dass die Betriebsinformation des Verdampfers eine perzeptive Information ist, die an den Benutzer gesendet wird, die mindestens: eine visuelle Information über einen Bildschirm, eine Leuchtanzeige, eine akustische Information über einen Warnton und eine taktile Information über eine Vibration einschließt.

12. Ein Verfahren zum Steuern der E-Zigarette nach einem der Ansprüche 7 bis 11, **dadurch gekennzeichnet, dass** das Verfahren die Schritte aufweist:
- des Erwerbens und des Aufzeichnens der Identifikationsinformationen, des Sendens der aufgezeichneten Identifikationsinformationen des Verdampfers an die Steuerung der E-Zigarette (201),
- des Empfangens der Identifikationsinformationen des Verdampfers, des Identifizierens und des Authentifizierens der empfangenen Identifikationsinformationen, des Steuerns des Betriebs des Verdampfers für E-Zigaretten, wenn das Identifizieren und das Authentifizieren gelungen sind, des Stoppens des Betriebs des Verdampfers für E-Zigaretten, wenn das Identifizieren und das Authentifizieren scheitern (202),
- des Erwerbens und des Aufzeichnens der Betriebsinformationen des Verdampfers, die von dem Aufzeichnungschip aufgezeichnet werden, des Sendens der Betriebsinformationen des Verdampfers an die Steuerung der E-Zigarette (203),
- des Empfangens der Betriebsinformationen des Verdampfers und des Bestimmens des eventuellen Überschreitens der Betriebsdauer des Verdampferkerns und des Stoppens des Betriebs des Verdampfers für E-Zigaretten, wenn der Verdampferkern seine Betriebsdauer (204) überschritten hat.

13. Das Verfahren zum Steuern der der E-Zigarette nach Anspruch 12, **dadurch gekennzeichnet**, welches Verfahren ferner umfasst dass beim Empfangen der Identifikationsinformationen des Verdampfers für E-Zigaretten das Vergleichen und das Verknüpfen der Identifikationsinformationen des Verdampfers mit den vordefinierten Identifikationsinformationen des Verdampfers für E-Zigaretten, wenn das Vergleichen und das Verknüpfen gelungen sind, in Abhängigkeit von den erhaltenen Identifikationsinformationen des Verdampfers für E-Zigaretten, die von dem Aufzeichnungschip empfangen und gesendet werden, das Einstellen der Betriebsleistung, des Stroms und der Temperatur des Verdampferkerns, um den Betrieb des Verdampfers für E-Zigaretten zu steuern.

14. Das Verfahren zum Steuern der E-Zigarette nach Anspruch 12 oder 13, **dadurch gekennzeichnet**, welches Verfahren ferner umfasst dass nach dem Empfangen der Betriebsinformationen des Verdampfers für E-Zigaretten, die von dem Aufzeichnungschip empfangen und gesendet werden, das Vergleichen mit den entsprechenden vordefinierten Schwellenwerten für Dauern und Schwellenwerten von einer Anzahl von Verwendungen, wenn sich die Betriebsinformationen des Verdampfers den vordefinierten Schwellenwerten der Betriebsdauern und/oder Schwellenwerten der Anzahl der Verwendungen annähern oder diese überschreiten, das Stoppen des Betriebs des Verdampfers für E-Zigaretten, wobei das Austauschen des Verdampferkerns vor dem Stoppen angezeigt wird.c

## Claims

1. An electronic cigarette atomizer, said electronic cigarette atomizer comprises an atomizer core for atomizing an aromatic liquid of the electronic cigarette, said electronic cigarette atomizer is equipped with a recording chip (1), said recording chip (1) is designed for:
- recording identification information of the electronic cigarette atomizer, said identification information comprising at least aromatic liquid information and atomizer core information, for sending said atomizer identification information to an electronic cigarette controller (5); and
- recording operating information of the electronic cigarette atomizer, said operating information including at least the service life and the number of uses of the atomizer core, and for sending said operating information of the atomizer to the electronic cigarette controller,
**characterized in that** said aromatic liquid information includes the composition of the aromatic liquid and the amount of aromatic liquid, and **in that** said atomizer core information includes the material of heating wires, the resistance values of the heating wires and the information concerning the aromatic liquid suitable for the atomizer core.

2. The electronic cigarette atomizer according to claim 1, **characterized in that** said recording chip (1) comprising:
- a first information recorder (2), for recording the identification information of the atomizer;
- a second information recorder (3), for recording the operation information of the atomizer; and
- an information transmitter (4) for sending the atomizer identification information and the operating information obtained from the atomizer to the electronic cigarette controller (5).

3. The electronic cigarette atomizer according to claim 2, **characterized in that** when said electronic cigarette atomizer is provided with an aromatic liquid cartridge, said aromatic liquid cartridge is provided with a first information recorder (2).

4. The electronic cigarette atomizer according to claim 3, **characterized in that** said first information recorder (2) comprises an information marker and an information identifier, said information marker is adapted to connect to said aromatic liquid cartridge, wherein when an aromatic liquid cartridge is installed with said electronic cigarette atomizer, said information identifier identifies said information marker, acquires the aromatic liquid cartridge identification information and, based on said aromatic liquid cartridge identification information, confirms and records the aromatic liquid information and the atomizer information.

5. The electronic cigarette atomizer according to claim 4, **characterized in that** said information marker is a barcode, a QR code or a magnetic stripe, said information marker is a corresponding identifier.

6. The electronic cigarette atomizer according to any one of claims 2 to 5, **characterized in that** said second information recorder (3) is connected to said atomizer core.

7. An electronic cigarette, **characterized in that** said electronic cigarette comprising an electronic cigarette atomizer equipped with the recording chip (1) according to any one of claims 1 to 6, and an electronic cigarette controller (5) designed for:
- receiving the atomizer identification information registered by said recording chip (1), identifying and authenticating the received atomizer identification information, checking the operation of the electronic cigarette atomizer when the identification and authentication is successful, stopping the operation of the electronic cigarette atomizer when the identification and authentication fails; and
- receiving atomizer operation information recorded by said recording chip (1), determining whether the atomizer core has exceeded its service life, and stopping the operation of the electronic cigarette atomizer when the atomizer core has exceeded its service life.

8. The electronic cigarette according to claim 7, **characterized in that** said electronic cigarette controller further comprises:
- an identifier-authenticator (6), for receiving the identification information of said electronic cigarette atomizer, for comparing and matching the identification information of the electronic cigarette atomizer with the predefined identification information of the electronic cigarette atomizer, when the comparison and matching are successful, adjusting the operating parameters of the atomizer core in accordance with the received electronic cigarette atomizer identification information, said operating parameters including at least the operating power, current and temperature of the atomizer core, in order to control the operation of the electronic cigarette atomizer.

9. The electronic cigarette according to claim 7 or 8, **characterized in that** said electronic cigarette controller further comprises: an operating time overrun controller (7) for acquiring atomizer operating information, comparing the atomizer operating information with predefined operating time threshold values and/or number of uses threshold values, and interrupting the operation of the electronic cigarette atomizer when the atomizer operating information exceeds the predefined operating time threshold values and/or number of uses threshold values.

10. The electronic cigarette according to claim 9, **characterized in that** said electronic cigarette is provided with a display, for displaying the identification information of the atomizer, when the identification information of the atomizer is identified and authenticated; and for displaying the operation information of the atomizer, indicating the replacement of the atomizer core, when the atomizer core approaches or exceeds its service life.

11. The electronic cigarette according to claim 10, **characterized in that** the operation information of the atomizer is sent before the operation of the atomizer of the electronic cigarette is interrupted; and **in that** the operation information of the atomizer is perceptual information sent to the user including at least: visual information via a screen, a warning light, sound information via a warning sound, and tactile information via a vibration.

12. A method of controlling the electronic cigarette according to any one of claims 7 to 11, **characterized in that** said method comprises the steps of:
- acquiring and recording atomizer identification information, sending the recorded atomizer identification information to the electronic cigarette controller (201);
- receiving said atomizer identification information, identifying and authenticating the received atomizer identification information, checking the operation of the electronic cigarette atomizer when the identification and authentication is successful, stopping the operation of the electronic cigarette atomizer when the identification and authentication is unsuccessful (202);
- acquiring and recording atomizer operation information recorded by the recording chip, sending said atomizer operation information to the electronic cigarette controller (203);
- receiving atomizer operating information and determining whether the service life of the atomizer core has been exceeded, and stopping the operation of the electronic cigarette atomizer when the atomizer core has exceeded its service life (204).

13. The method of controlling the electronic cigarette according to claim 12, **characterized in that** the method further comprises, upon receiving the identification information of said electronic cigarette atomizer, comparing and matching the atomizer identification information with predefined electronic cigarette atomizer identification information, when the comparison and matching are successful, in accordance with the electronic cigarette atomizer identification information received and sent from said recording chip, adjusting the operating power, current and temperature of the atomizer core to control the operation of the electronic cigarette atomizer.

14. The method of controlling the electronic cigarette according to claim 12 or 13, **characterized in that** the method further comprises, after receiving the operating information of the electronic cigarette atomizer received and sent by said recording chip, comparing with the corresponding predefined duration threshold values and number of use threshold values, when the operating information of the atomizer approaches or exceeds the predefined duration threshold values and/or number of use threshold values, stopping the operation of the electronic cigarette atomizer, replacing the atomizer core is indicated before stopping.
